# EUROPEAN PATENT APPLICATION

(11) **EP 4 810 549 A1**
(43) Date of publication of application: **23.09.2026**
(21) Application number: 24907246.3
(22) Date of filing: 10.12.2024
(51) Int. Cl.: C08J 11/24, C07C 27/02, C07C 29/128, C07C 31/20, C07C 67/03, C07C 69/82

(54) **REACTOR AND MONOMER PRODUCTION SYSTEM**

(30) Priority: 20.12.2023 JP 2023214807
(71) Applicant: Mitsubishi Heavy Industries, Ltd., Chiyoda-ku Tokyo 100-8332 (JP)
(72) Inventor: MATSUBARA, Wataru, Tokyo 100-8332 (JP); SEIKI, Yoshio, Tokyo 100-8332 (JP); MIYATA, Yasuyuki, Tokyo 100-8332 (JP); GENTA, Minoru, Tokyo 100-8332 (JP); ITO, Motofumi, Tokyo 100-8332 (JP)
(74) Representative: Studio Torta S.p.A.
(86) International application number: PCT/JP2024/043576
(87) International publication number: WO 2025/134861

(57) **Abstract**

To suppress a decrease in yield of monomer. A reactor is a reactor into which polyester and a reaction solvent that reacts with the polyester are introduced to depolymerize the polyester, and includes: a reaction section that includes a distribution section that includes first flow paths extending from an introduction port into which a reaction solvent with a first depolymerized polyester as depolymerized polyester extracted therefrom is introduced to a lead-out port from which a reaction solvent with a second depolymerized polyester as the further depolymerized first depolymerized polyester extracted therefrom is led out and a resistor that is provided at a position at which the resistor blocks the lead-out port of the distribution section, second flow paths through which the reaction solvent flowing through the first flow paths is allowed to flow being formed in the resistor, the resistor having a higher pressure loss than the distribution section.

## Description

### Field

The present disclosure relates to a reactor and a monomer production system.

### Background

In recycling of polyester, a technique of monomerizing polyester by depolymerization (reverse reaction of polymerization) is known. Patent Literature 1 describes a reaction vessel including: a first reaction section that causes a polyester raw material to react with a reaction solvent; and a second reaction section that causes a low-molecular-weight polyester obtained in the first reaction section to further react with a reaction solvent from which a monomer has been extracted to form a monomer. Patent Literature 1 describes that drift of the reaction solvent can be suppressed by providing a rectifying means for rectifying the reaction solvent in the second reaction section.

### Citation List

### Patent Literature

Patent Literature 1: JP 2005-289826 A

### Summary

### Technical Problem

However, there is a concern that convection may occur in the second reaction section due to unevenness in temperature distribution of the reaction solvent, and the retention time of the reaction solvent may be shortened, leading to a decrease in yield of the monomer.

An object of the present disclosure is to provide a reactor and a monomer production system capable of suppressing a decrease in yield of a monomer.

### Solution to Problem

A reactor according to the present disclosure is a reactor into which polyester and a reaction solvent that reacts with the polyester are introduced to depolymerize the polyester, and includes a reaction section. The reaction section includes: a distribution section that includes a plurality of first flow paths extending from an introduction port to a lead-out port, the reaction solvent with a first depolymerized polyester as the depolymerized polyester extracted therefrom being introduced into the introduction port, the reaction solvent with a second depolymerized polyester as the further depolymerized first depolymerized polyester extracted therefrom being led out from the lead-out port; and a resistor that is provided at a position at which the resistor blocks the lead-out port of the distribution section, second flow paths through which the reaction solvent flowing through the first flow paths is allowed to flow being formed in the resistor. The resistor has a higher pressure loss than the distribution section.

A monomer production system according to the present disclosure includes: the above-described reactor; and a separation section that is connected to the reactor and separates the reaction solvent with the second depolymerized polyester dissolved therein into the reaction solvent, a monomer derived from a carboxylic acid contained in the second depolymerized polyester, and a monomer of an alcohol component contained in the second depolymerized polyester.

### Advantageous Effects of Invention

According to the present disclosure, a decrease in yield of a monomer can be suppressed.

### Brief Description of Drawings

FIG. 1 is a schematic view of a polyester recycling step in the present embodiment.
FIG. 2 is a schematic view of a monomer production system according to the present embodiment.
FIG. 3 is a schematic view of a second reaction section.
FIG. 4 is a schematic top view of a distribution section.

### Description of Embodiments

Hereinafter, a preferred embodiment of the present disclosure will be described in detail with reference to the accompanying drawings. Note that the present disclosure is not limited by the embodiment, and in a case where there are a plurality of embodiments, the present disclosure also includes configurations achieved by combining the embodiments.

### (Recycling Step)

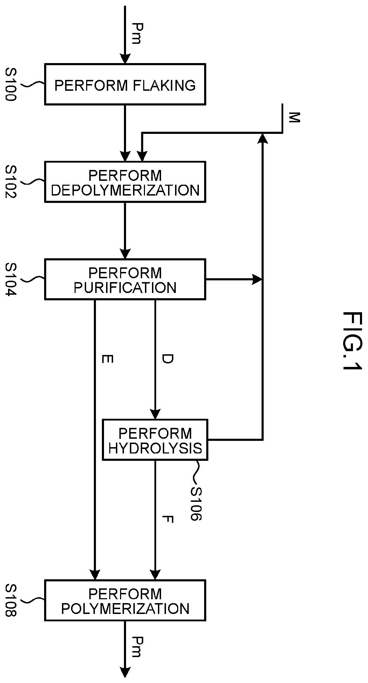

FIG. 1 is a schematic view of a polyester recycling step in the present embodiment. In the present embodiment, a step of recycling (regenerating) a polyester raw material Pm is performed by depolymerizing the polyester raw material Pm to form a monomer and polymerizing the monomer again. Specifically, as illustrated in FIG. 1, the polyester raw material Pm is flaked (Step S100), the flaked polyester raw material Pm is dissolved in a monomer D derived from a carboxylic acid to produce a polyester solution (Step S101), foreign substances are removed from the polyester solution (Step S102), the polyester solution with the foreign substances removed therefrom is mixed with a reaction solvent M to depolymerize the polyester solution (Step S103), the monomer of the depolymerized polyester is purified (separated) to produce the monomer D derived from the carboxylic acid and a monomer E of an alcohol component (Step S104), the monomer D is hydrolyzed to separate the reaction solvent M (Step S106), and a monomer F produced through the hydrolysis of the monomer D and the monomer E are polymerized (Step S108) to reproduce the polyester raw material Pm. Note that in the recycling step in which the monomer production system 1 of the present embodiment is adopted, the flaking in Step S100 may be omitted, or only the treatment of recovering the monomers D and E illustrated in Step S102 and Step S104 and the monomer F illustrated in Step S106 may be performed without performing up to the repolymerizing treatment as in Step S108.

### (Polyester Raw Material)

The polyester raw material Pm to be depolymerized in the present embodiment is a substance containing polyester. Although the polyester raw material Pm is not particularly limited, examples thereof include waste products such as polyethylene terephthalate (PET), polyethylene butylene terephthalate (PEBT), polybutylene terephthalate (PBT), polycyclohexanedimethyl terephthalate (PCT), polyethylene naphthalate (PEN), polybutylene naphthalate (PBN), and polycarbonate (PC). The polyester raw material Pm is not limited to one containing only a polyester component and may also contain components (impurities) other than the polyester component. Examples of the components other than polyester contained in the polyester raw material Pm include plastics other than polyester, such as polyethylene, polystyrene, polypropylene, and polyvinyl chloride, metals, dyes, pigments, and polymerization catalysts. Examples of the polyester raw material Pm also include clothing in which polyester and other components are woven into a fibrous form.

### (Reaction Solvent)

The reaction solvent M is a solvent that reacts with polyester to depolymerize the polyester. The reaction solvent M may be, for example, at least one of methanol, ethanol, water, and ethylene glycol.

### (Monomer Derived from Carboxylic Acid)

The monomer D derived from a carboxylic acid is a monomer having a carboxyl group produced through a depolymerization reaction of polyester. The monomer D may be, for example, dimethyl carboxylate or diethyl carboxylate. Furthermore, the monomer D is preferably a monomer of a terephthalic acid, and may be, for example, dimethyl terephthalate (DMT).

### (Monomer of Alcohol Component)

The monomer E of an alcohol component is a monomer of an alcohol component produced through a depolymerization reaction of polyester. The monomer E may be, for example, a dihydroxy compound (dihydric alcohol), more specifically, ethylene glycol (EG).

Hereinafter, a case where the polyester is PET, the reaction solvent M is methanol, the monomer D is DMT, and the monomer E is EG will be described as an example.

### (Monomer Production System)

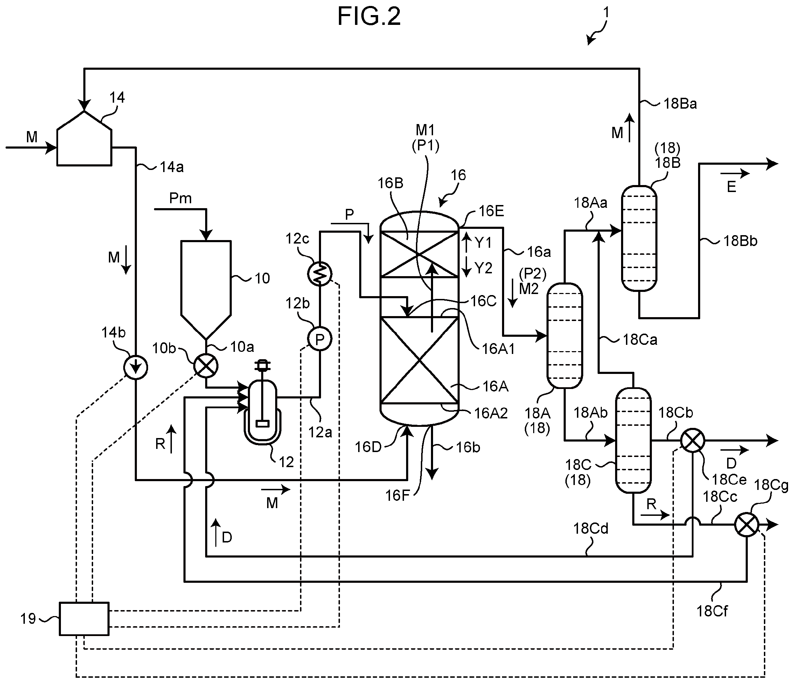

FIG. 2 is a schematic view of a monomer production system according to the present embodiment. A monomer production system 1 according to the present embodiment is a system that monomerizes polyester contained in the polyester raw material Pm to produce the monomers D and E. As illustrated in FIG. 2, the monomer production system 1 includes a raw material storage section 10, a dissolution section 12, a solvent storage section 14, a reactor 16, a separation section 18, and a control section 19.

### (Raw Material Storage Section)

The raw material storage section 10 is a tank into which the polyester raw material Pm is introduced and in which the polyester raw material Pm is stored. Although the flaked polyester raw material Pm is stored in the raw material storage section 10 in the present embodiment, the shape and the size of the polyester raw material Pm may be arbitrary. The raw material storage section 10 is connected to the dissolution section 12 via an introduction pipe 10a. The polyester raw material Pm in the raw material storage section 10 is supplied to the dissolution section 12 through the introduction pipe 10a. The introduction pipe 10a is provided with an adjustment section 10b that adjusts the amount of polyester raw material Pm to be supplied from the raw material storage section 10 to the dissolution section 12. The adjustment section 10b is, for example, an opening/closing valve, and supplies the polyester raw material Pm in the raw material storage section 10 to the dissolution section 12 in an open state, and stops the supply of the polyester raw material Pm in the raw material storage section 10 to the dissolution section 12 in a closed state. However, the adjustment section 10b is not limited to the opening/closing valve and may be any mechanism capable of adjusting the supply of the polyester raw material Pm to the dissolution section 12. Also, the polyester raw material Pm may be supplied directly to the dissolution section 12 without passing through the raw material storage section 10, the introduction pipe 10a, and the adjustment section 10b.

### (Dissolution Section)

The dissolution section 12 is a tank in which the polyester solution P is stored. The polyester solution P is a solution in which the polyester contained in the polyester raw material Pm is dissolved in the monomer D. The polyester solution P is a solution produced by the polyester raw material Pm and the monomer D being mixed. The monomer D and the polyester raw material Pm are supplied to the dissolution section 12, and the polyester contained in the polyester raw material Pm is dissolved in the monomer D in the dissolution section 12 to produce the polyester solution P. The viscosity can be reduced, fluidity can be improved, and polyester can be easily led out to the reactor 16, by dissolving polyester in the monomer D in this manner. More specifically, a residual substance R, which will be described later, is also supplied to the dissolution section 12, and the polyester contained in the polyester raw material Pm is dissolved in the monomer D and the oligomer contained in the residual substance R in the dissolution section 12 to produce the polyester solution P. In other words, it can be said that the polyester solution P is a solution of polyester dissolved in the monomer D and the residual substance R. However, the polyester solution P is not limited to a solution in which the total amount of polyester is dissolved in the monomer D and the residual substance R, and may be in a state where at least a part of polyester is not dissolved in the monomer D and the residual substance R. The polyester raw material Pm may contain impurities which are substances other than polyester. In this case, it can be said that the polyester solution P contains the monomer D, the dissolved polyester, and the impurities.

Note that the polyester solution P is not limited to a solution in which polyester is dissolved in the monomer D, and may be a solution in which polyester is dissolved in the monomer E. In this case, the monomer E and the polyester raw material Pm are supplied to the dissolution section 12, and polyester contained in the polyester raw material Pm is dissolved in the monomer E in the dissolution section 12 to produce the polyester solution P.

The dissolution section 12 is connected to a first reaction section 16A, which will be described later, via an introduction pipe 12a. The polyester solution P in the dissolution section 12 is supplied to the first reaction section 16A through the introduction pipe 12a. Also, the introduction pipe 12a is provided with a supply section 12b and a heating section 12c. The supply section 12b is a mechanism for supplying the polyester solution P in the dissolution section 12 to the first reaction section 16A, and is a pump in the present embodiment. The heating section 12c is a mechanism for heating the polyester solution P.

### (Solvent Storage Section)

The solvent storage section 14 is a tank into which the reaction solvent M is introduced and in which the reaction solvent M is stored. The solvent storage section 14 is connected to the reactor 16 via an introduction pipe 14a. The reaction solvent M in the solvent storage section 14 is supplied to the reactor 16 through the introduction pipe 14a. More specifically, the introduction pipe 14a is provided with a heating and pressure raising section 14b that pressurizes and heats the reaction solvent M. The heating and pressure raising section 14b pressurizes and heats the reaction solvent M to bring the reaction solvent M into a supercritical state or a subcritical state (pressurized gas or pressurized liquid). The reaction solvent M in the supercritical state or the subcritical state (pressurized gas or pressurized liquid) is supplied to the reactor 16.

### (Reactor)

The reactor 16 is a mechanism into which polyester and the reaction solvent M that reacts with the polyester are introduced to depolymerize the polyester. Specifically, the reactor 16 is a container into which the polyester solution P and the reaction solvent M are supplied and in which the polyester in the polyester solution P is depolymerized with the supplied reaction solvent M. The reactor 16 includes a first reaction section 16A as a pre-reaction section and a second reaction section 16B as a reaction section. Hereinafter, a direction directed from the first reaction section 16A to the second reaction section 16B in the reactor 16 is defined as a direction Y1, and the direction opposite to the direction Y1 (a direction directed from the second reaction section 16B to the first reaction section 16A) is defined as a direction Y2. In the present embodiment, the direction Y2 is the direction of gravity (downward in the vertical direction).

### (First Reaction Section)

The first reaction section 16A is formed inside the reactor 16. In the present embodiment, it can be said that the first reaction section 16A is a location filled with a filler inside the reactor 16. For the first reaction section 16A, a known filler used in a gas-liquid or liquid-liquid contact device can be used as a filler, and for example, a filler similar to a filler used in a contact device for taking out an active ingredient by bringing heavy oil into contact with water can be used. Specific examples of the filler include a pipe made of SUS or the like, Raschig rings, Berl saddles, and Tellerettes.

The introduction pipe 12a is connected to the first reaction section 16A. More specifically, an introduction port 16C as an opening of the introduction pipe 12a through which the polyester solution P from the dissolution section 12 is introduced is connected to the first reaction section 16A. The introduction port 16C is connected to the surface 16A1 of the first reaction section 16A on the direction Y1 side. The introduction pipe 12a is connected to the surface 16A1 such that the introduction port 16C opens toward the direction Y2 side. Although the introduction port 16C opening toward the direction Y2 side is connected to the surface 16A1 of the first reaction section 16A in the present embodiment in this manner, the present disclosure is not limited thereto. For example, the introduction port 16C is not necessarily connected directly to the first reaction section 16A, and the introduction port 16C opening toward the direction Y2 side may be connected to the direction Y1 side as compared with the surface 16A1 of the first reaction section 16A inside the reactor 16.

The introduction pipe 14a is connected to the reactor 16. More specifically, an introduction port 16D as an opening of the introduction pipe 14a through which the reaction medium M from the solvent storage section 14 is introduced is connected to the reactor 16. The introduction port 16D is connected to the direction Y2 side as compared with the surface 16A2 of the first reaction section 16A on the direction Y2 side. The introduction pipe 14a is connected on the direction Y2 side as compared with the surface 16A2 such that the introduction port 16D opens toward the direction Y1 side or from a side surface toward a center side. Although the introduction port 16D opening toward the direction Y1 side or from the side surface toward the center side is connected on the direction Y2 side as compared with the surface 16A2 of the first reaction section 16A in the present embodiment in this manner, the present disclosure is not limited thereto. For example, the introduction port 16D may be connected directly to the first reaction section 16A or may be connected to the surface 16A2 of the first reaction section 16A.

As described above, in the present embodiment, the introduction port 16C into which the polyester solution P is introduced opens toward the direction Y2, and the introduction port 16D into which the reaction solvent M is introduced opens toward the direction Y1 or from the side surface toward the center side. Therefore, the polyester solution P and the reaction solvent M are introduced into the first reaction section 16A in mutually facing directions.

The polyester solution P introduced from the introduction port 16C into the first reaction section 16A moves in the direction Y2 on the surface of the filler of the first reaction section 16A. On the other hand, the reaction solvent M in the supercritical state or the subcritical state (pressurized gas or pressurized liquid) introduced from the introduction port 16D moves in the direction Y1 in the first reaction section 16A. The reaction solvent M in the supercritical state or the subcritical state (pressurized gas or pressurized liquid) comes into contact with the polyester solution P in the first reaction section 16A. The polyester in the polyester solution P is depolymerized (reduced in molecular weight) with the reaction solvent M, and the depolymerized polyester is extracted into the reaction solvent M in the supercritical state or the subcritical state (pressurized gas or pressurized liquid). Hereinafter, the polyester depolymerized in the first reaction section 16A will be described as first depolymerized polyester P1, and a mixture of the first depolymerized polyester P1 and the reaction solvent M (the reaction solvent M in which the first depolymerized polyester P1 has been extracted) will be described as a first solvent M1. The first solvent M1 containing the first depolymerized polyester P1 advances through the first reaction section 16A toward the direction Y1 side and is then led out to the direction Y1 side of the first reaction section 16A.

Note that the first depolymerized polyester P1 includes monomers D and E produced by depolymerizing the polyester in the polyester solution P, a monomer D originally mixed in the polyester solution P, and an oligomer produced by depolymerizing the polyester. It can be said that the oligomer described here is an oligomer of a carboxylic acid or an alcohol (an oligomer of a carboxylic acid or an alcohol having a smaller molecular weight than polyester) depolymerized from polyester although it has not been monomerized. The oligomer contained in the residual substance R in the polyester solution P is also depolymerized with the reaction solvent M. Therefore, the first depolymerized polyester P1 also contains the depolymerized residual substance R. The depolymerized residual substance R is an oligomer contained in the residual substance R and depolymerized, or the monomers D, E, and the like produced by depolymerizing the oligomer contained in the residual substance R.

### (Second Reaction Section)

The second reaction section 16B is provided in the reactor 16 and is provided at a location where the first solvent M1 is led out from the first reaction section 16A. In the present embodiment, the second reaction section 16B is provided on the first direction D1 side of the first reaction section 16A.

In the second reaction section 16B, the first depolymerized polyester P1 contained in the first solvent M1 is further depolymerized (reduced in molecular weight) with the reaction solvent M contained in the first solvent M1. Hereinafter, the first depolymerized polyester P1 further depolymerized in the second reaction section 16B will be described as a second depolymerized polyester P2, and the mixture of the second depolymerized polyester P2 and the reaction solvent M (the reaction solvent M containing the second depolymerized polyester PE) will be described as a second solvent M2. The lead-out pipe 16a is connected to the second reaction section 16B. More specifically, a lead-out port 16E as an opening of the lead-out pipe 16a through which the second solvent M2 from the second reaction section 16B is led out is connected to the second reaction section 16B. The second solvent M2 containing the second depolymerized polyester P2 in the second reaction section 16B is led out from the lead-out port 16E to the outside of the second reaction section 16B through the lead-out pipe 16a.

Note that the second depolymerized polyester P2 contains the monomers D and E produced by depolymerizing the oligomer in the first depolymerized polyester P1 and the oligomer produced by depolymerizing the first depolymerized polyester P1.

A detailed structure of the second reaction section 16B will be described later.

A discharge pipe 16b is connected to a bottom portion (bottom surface on the direction Y2 side) of the reactor 16. More specifically, a discharge port 16F as an opening of the discharge pipe 16b through which non-extracted substances in the reactor 16 are discharged is connected to the bottom portion of the reactor 16. Non-extracted substances containing impurities such as metal compounds that have not been extracted to the reaction solvent M, residues of undecomposed polyester that has not been extracted to the reaction solvent M, and the like are discharged from the discharge pipe 16b. In other words, the non-extracted substances at the bottom portion of the reactor 16 are discharged from the discharge port 16F to the outside of the reactor 16 through the discharge pipe 16b. It can be said that the non-extracted substances discharged from the discharge pipe 16b are components remaining in the first reaction section 16A and the second reaction section 16B without being led out to the separation section 18 as the second solvent M2 (the reaction solvent M containing the second depolymerized polyester P2) in the polyester solution P.

The reactor 16 may be provided with a heating section that heats the inside of the reactor 16 and a pressurizing section that keeps the pressure inside the reactor 16 at a predetermined value or more. The temperature inside the reactor 16 is preferably equal to or greater than 250°C and equal to or less than 400°C, and is more preferably equal to or greater than 250°C and equal to or less than 350°C. The pressure inside the reactor 16 is preferably equal to or greater than 1 MPa and equal to or less than 30 MPa, and is more preferably equal to or greater than 6 MPa and equal to or less than 25 MPa. The pressurizing section and the heating section may be controlled by the control section 19.

### (Separation Section)

The second solvent M2 containing the second depolymerized polyester P2 is introduced into the separation section 18, and the separation section 18 separates the second solvent M2 into the reaction solvent M, the monomer D derived from a carboxylic acid contained in the second depolymerized polyester P2, the monomer E of an alcohol component contained in the second depolymerized polyester P2, and the residual substance R. The residual substance R is a component other than the reaction solvent M, the monomer D, and the monomer E in the second solvent M2, and contains an oligomer.

In the present embodiment, the separation section 18 includes a first separation section 18A, a second separation section 18B, and a third separation section 18C.

The first separation section 18A is a separation tower connected to the lead-out pipe 16a. The second solvent M2 containing the second depolymerized polyester P2 is introduced into the first separation section 18A via the lead-out pipe 16a. The first separation section 18A separates the second solvent M2 into a low-boiling-point component and a high-boiling-point component having a higher boiling point than the low-boiling-point component. For example, the second solvent M2 may be set to a predetermined temperature, a component in the form of gas may be regarded as the low-boiling-point component, and a liquid component may be regarded as the high-boiling-point component, in the first separation section 18A. Lead-out pipes 18Aa and 18Ab are connected to the first separation section 18A. The low-boiling-point component is led out from the lead-out pipe 18Aa, and the high-boiling-point component is led out from the lead-out pipe 18Ab.

The second separation section 18B is a separation tower connected to the first separation section 18A via the lead-out pipe 18Aa. The low-boiling-point component is introduced into the second separation section 18B via the lead-out pipe 18Aa. The second separation section 18B separates the low-boiling-point component into the reaction solvent M and the monomer E. Lead-out pipes 18Ba and 18Bb are connected to the second separation section 18B. The reaction solvent M is led out from the lead-out pipe 18Ba, and the monomer E is led out from the lead-out pipe 18Bb. Note that the lead-out pipe 18Ba is connected to the second separation section 18B and the solvent storage section 14. Therefore, the reaction solvent M led out from the second separation section 18B is returned to the solvent storage section 14 and is then reused for monomerization of polyester.

The third separation section 18C is a separation tower connected to the first separation section 18A via the lead-out pipe 18Ab. The high-boiling-point component is introduced into the third separation section 18C via the lead-out pipe 18Ab. The third separation section 18C further separates the high-boiling-point component into a high-boiling-point residual substance R, a low-boiling-point component including the reaction solvent M and the monomer E, and the monomer D. Lead-out pipes 18Ca, 18Cb, and 18Cc are connected to the third separation section 18C. The lead-out pipe 18Ca is connected to the second separation section 18B. The low-boiling-point component separated inside the third separation section 18C is led out to the second separation section 18B via the lead-out pipe 18Ca. Also, the monomer D separated inside the third separation section 18C is led out from the lead-out pipe 18Cb, and the residual substance R separated inside the third separation section 18C is led out from the lead-out pipe 18Cc.

An introduction pipe 18Cd is connected to the third separation section 18C. The introduction pipe 18Cd is also connected to the dissolution section 12 and introduces the monomer D led out from the third separation section 18C into the dissolution section 12. In the example of FIG. 2, the introduction pipe 18Cd is branched from the lead-out pipe 18Cb. The introduction pipe 18Cd is provided with an adjustment section 18Ce that adjusts the amount of monomer D to be supplied from the third separation section 18C to the dissolution section 12. The adjustment section 18Ce is, for example, an opening/closing valve, causes the monomer D to be supplied to the dissolution section 12 in the open state, and causes the supply of the monomer D to the dissolution section 12 to be stopped in a closed state. However, the adjustment section 18Ce is not limited to the opening/closing valve and may be any mechanism capable of adjusting the supply of the monomer D to the dissolution section 12. Note that although the adjustment section 18Ce is provided at the branched location of the introduction pipe 18Cd from the lead-out pipe 18Cb in the present embodiment, the position where the adjustment section 18Ce is provided is not limited thereto and may be any position. Also, the introduction pipe 18Cd is not necessarily connected to the lead-out pipe 18Cb, and may be connected directly to the third separation section 18C. For example, a storage section (tank) for storing the monomer D may be provided in the lead-out pipe 18Cb, and the introduction pipe 18Cd may be connected to the storage section.

An introduction pipe 18Cf is connected to the third separation section 18C. The introduction pipe 18Cf is also connected to the dissolution section 12 and introduces the residual substance R led out from the third separation section 18C into the dissolution section 12. In the example of FIG. 2, the introduction pipe 18Cf is branched from the lead-out pipe 18Cc. The introduction pipe 18Cf is provided with an adjustment section 18Cg that adjusts the amount of residual substance R to be supplied from the third separation section 18C to the dissolution section 12. The adjustment section 18Cg is, for example, an opening/closing valve, causes the residual substance R to be supplied to the dissolution section 12 in an open state, and causes the supply of the residual substance R to the dissolution section 12 to be stopped in a closed state. However, the adjustment section 18Cg is not limited to the opening/closing valve and may be any mechanism capable of adjusting the supply of the residual substance R to the dissolution section 12. Note that although the adjustment section 18Cg is provided at the branched location of the introduction pipe 18Cf from the lead-out pipe 18Cc in the present embodiment, the position where the adjustment section 18Cg is provided is not limited thereto and may be any position. Also, the introduction pipe 18Cf is not necessarily connected to the lead-out pipe 18Cc, and may be connected directly to the third separation section 18C.

Note that the lead-out pipe 18Cc may be provided with a storage section (tank) for storing the residual substance R, and the introduction pipe 18Cf may be connected to the storage section, for example. The introduction pipe 18Cf may be provided with a filter that collects foreign substances in the residual substance R while allowing the oligomer in the residual substance R to pass therethrough.

In the example of FIG. 2, the introduction pipe 10a, the introduction pipe 18Cd, and the introduction pipe 18Cf connected to the dissolution section 12 are not connected to each other and are connected directly to the dissolution section 12. However, at least two of the introduction pipe 10a, the introduction pipe 18Cd, and the introduction pipe 18Cf may be connected (merged), and the connected pipes may then be connected to the dissolution section 12.

Note that since the polyester solution P is a solution of polyester dissolved in the monomer D in the present embodiment, the configuration in which the monomer D is introduced into the dissolution section 12 via the introduction pipe 18Cd is adopted. However, in a case where the polyester solution P is a solution of polyester dissolved in the monomer E, the introduction pipe 18Cd may be connected to the second separation section 18B and the dissolution section 12. In other words, the monomer E separated inside the second separation section 18B is introduced into the dissolution section 12 via the introduction pipe 18Cd in this case. Note that a storage section (tank) for storing the monomer E may be provided and the introduction pipe 18Cd may be connected to the storage section.

### (Control Section)

The control section 19 is a control device that controls the monomer production system 1. The control section 19 controls the adjustment section 10b to control the amount of polyester raw material Pm to be supplied from the raw material storage section 10 to the dissolution section 12. The control section 19 controls the supply section 12b to control the amount of polyester solution P to be supplied from the dissolution section 12 to the first reaction section 16A. The control section 19 controls the heating section 12c to control the degree of heating of the polyester solution P. The control section 19 controls the heating and pressure raising section 14b, brings the reaction solvent M into the supercritical state or the subcritical state (pressurized gas or pressurized liquid), and controls the amount of reaction solvent M in the supercritical state or the subcritical state (pressurized gas or pressurized liquid) to be supplied to the reactor 16. The control section 19 controls the adjustment section 18Ce to control the amount of monomer D to be supplied to the dissolution section 12. The control section 19 controls the adjustment section 18Cg to control the amount of residual substance R to be supplied to the dissolution section 12.

The control section 19 is a computer in the present embodiment and includes, for example, a processor including an arithmetic circuit such as a central processing unit (CPU) and a storage section that stores various kinds of information such as arithmetic content of the processor, programs, and the like. The control section 19 executes control of the monomer production system 1 by reading the programs from the storage section.

However, the monomer production system 1 is not limited to being automatically controlled by the control section 19, and at least a part of processing may be controlled by an operator's operation, for example.

### (Operations of Monomer Production System)

Next, operations of the monomer production system 1 will be described. The control section 19 introduces the polyester raw material Pm, the monomer D, and the residual substance R into the dissolution section 12 to produce the polyester solution P. Then, the control section 19 introduces the polyester solution P and the reaction solvent M into the first reaction section 16A to depolymerize them and extracts the first depolymerized polyester P1 into the reaction solvent M. Then, the first solvent M1 in which the first depolymerized polyester P1 has been extracted is introduced into the second reaction section 16B, and the first depolymerized polyester P1 is further depolymerized in the second reaction section 16B to produce the second depolymerized polyester P2. Then, the second solvent M2 with the second depolymerized polyester P2 dissolved therein is separated into the reaction solvent M, the monomer D, the monomer E, and the residual substance R in the separation section 18.

### (Configuration of Second Reaction Section)

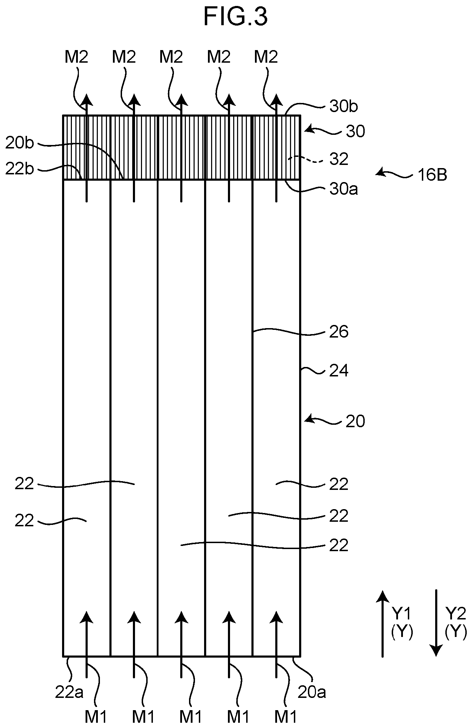

Next, a configuration of the second reaction section 16B will be described. FIG. 3 is a schematic view of the second reaction section. As illustrated in FIG. 3, the second reaction section 16B includes a distribution section 20 and a resistor 30.

### (Distribution Section)

The distribution section 20 is a member including a plurality of first flow paths 22 extending from introduction ports 22a to lead-out ports 22b. The introduction ports 22a are openings of the first flow paths 22 on the Y2 direction side, and the lead-out ports 22b are openings of the first flow path 22 on the Y1 direction side. The first flow paths 22 are flow paths that establish communication between the introduction ports 22a and the lead-out ports 22b, and in the present embodiment, the first flow paths 22 extend in the Y1 direction from the introduction ports 22a to the lead-out ports 22b. In other words, the distribution section 20 extends in the Y1 direction. The first flow paths 22 are provided to be aligned in the direction perpendicularly intersecting the Y1 direction (Y2 direction). In other words, it can be said that an introduction port 20a as an opening of the distribution section 20 on the Y1 direction side is constituted by the introduction ports 22a of the first flow paths 22 and a lead-out port 20b as an opening of the distribution section 20 on the Y2 direction side is constituted by the lead-out ports 22b of the first flow paths 22. Note that the number and the size of the first flow paths 22 may be any number and size.

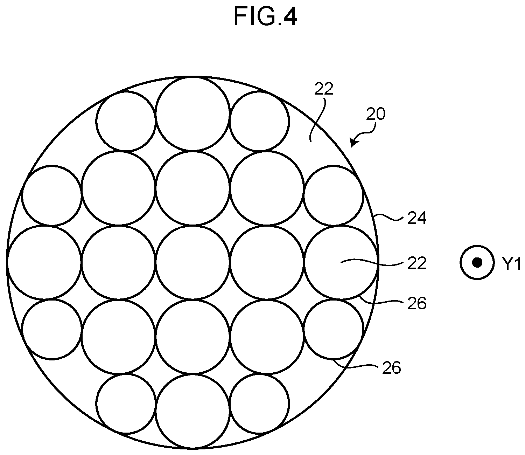

FIG. 4 is a schematic top view of the distribution section. As illustrated in FIG. 4, the distribution section 20 includes an outer pipe 24 and a plurality of inner pipes 26 in the present embodiment. The outer pipe 24 is a tubular member extending in the Y1 direction. Although the side wall of the reactor 16 constitutes the outer pipe 24 in the present embodiment, the outer pipe 24 may be provided on the side further inward than the side wall of the reactor 16. The inner pipes 26 are tubular members provided inside the outer pipe 24 and extending in the Y1 direction. The inner pipes 26 are provided to be aligned in the direction perpendicularly intersecting the Y1 direction (Y2 direction). In the present embodiment, a space inside each inner pipe 26 and the spaces surrounded by the inner wall of the outer pipe 24 and the outer walls of the inner pipes 26 form the first flow paths 22. Note that the number and arrangement of the inner pipes 26 are not limited to those in the example of FIG. 4 and may be any number and arrangement.

The first solvent M1 containing the first depolymerized polyester P1 led out from the first reaction section 16A is introduced into each first flow path 22 from the introduction port 20a of the distribution section 20 (the introduction port 22a of each first flow path 22). With the first solvent M1 introduced into the first flow paths 22 flowing toward the lead-out port 20b of the distribution section 20 (the lead-out port 22b of each first flow path 22), the first depolymerized polyester P1 contained in the first solvent M1 is further depolymerized with the reaction solvent M, and the resultant is led out as the second solvent M2 containing the second depolymerized polyester P2 from the lead-out port 20b (the lead-out port 22b of each first flow path 22).

### (Resistor)

The resistor 30 is a member through which the second solvent M2 flowing through the distribution section 20 (first flow paths 22) is allowed to flow and which has a higher pressure loss than the distribution section 20. The high pressure loss means that in a case where the same flow amount of fluid is caused to flow, the pressure loss of the fluid is higher than a comparison target. In other words, it can be said that the pressure loss of the fluid when the fluid is introduced from an end surface 30a of the resistor 30 and the fluid is led out from an end surface 30b is higher than the pressure loss when the same amount of fluid is introduced from the introduction port 20a of the distribution section 20 and the fluid is led out from the lead-out port 20b. Note that the resistor 30 preferably has a higher pressure loss than the first reaction section 16A.

The resistor 30 is provided at a position at which the lead-out port 20b of the distribution section 20 is blocked. The position at which the lead-out port 20b is blocked indicates a position between the introduction port 20a and the lead-out port 20b inside the distribution section 20 or a position at which the resistor 30 covers the lead-out port 20b opening on the direction Y1 side. Although the resistor 30 may be provided at any position at which the resistor 30 blocks the lead-out port 20b of the distribution section 20, the resistor 30 is provided at a position facing the lead-out port 20b of the distribution section 20 (the position at which the resistor 30 covers the lead-out port 20b) in the present embodiment. In other words, the resistor 30 is placed on the end surface (the end surfaces of the inner pipes 26 and the outer pipe 24 in the Y1 direction in this example) of the distribution section 20 in the Y1 direction and is in contact with the end surface of the distribution section 20 in the Y1 direction. However, the present disclosure is not limited thereto, and the resistor 30 may be provided inside the first flow paths 22 of the distribution section 20.

In addition, it is desired that the resistor 30 block the lead-out ports 22b of all the first flow paths 22 in the distribution section 20. In other words, the resistor 30 is preferably provided to block the entire region (the entire region of the lead-out ports 22b of all the first flow paths 22) of the lead-out port 20b of the distribution section 20.

Second flow paths 32 through which the second solvent M2 flowing through the first flow path 22 is allowed to flow are formed in the resistor 30. In other words, the resistor 30 increases the pressure loss of the second solvent M2 while allowing the second solvent M2 to flow therethrough by the second flow paths 32 being formed. The second flow paths 32 are flow paths that establish communication between the end surface 30a of the resistor 30 on the Y2 direction side and the end surface 30b of the resistor 30 on the Y1 direction side. The plurality of second flow paths 32 are provided inside the resistor 30.

The second flow paths 32 may have any shape that allows the second solvent M2 to have a higher pressure loss than the distribution section 20 while establishing communication between the end surface 30a and the end surface 30b. For example, the second flow paths 32 preferably extend so as not to follow the Y1 direction from openings on the end surface 30b side to the openings on the end surface 30a side. When the length from the end surface 30b to the end surface 30a in the Y1 direction is assumed to be a length L, for example, the flow path length from the openings of the second flow paths 32 on the end surface 30b side to the openings on the end surface 30a side is preferably longer than the length of the first flow paths 22 in the section of the length L in the Y1 direction. As a result, the pressure loss of the resistor 30 can be appropriately increased.

Note that although the resistor 30 may be any structure in which the second flow paths 32 are formed, the resistor 30 may be, for example, a porous body, a network structure, or the like, and a sintered filter is suitably used.

The pressure loss of the resistor 30 is preferably equal to or greater than 50 Pa, and is more preferably equal to or greater than 75 Pa and equal to or less than 125 Pa. Also, the ratio of the pressure loss of the resistor 30 to the pressure loss of the distribution section 20 is preferably equal to or greater than 110%, and is preferably equal to or greater than 200%. Acceleration of flows of the first solvent M1 and the second solvent M2 can be appropriately suppressed, and the decrease in yield of monomer can be appropriately suppressed by the pressure loss of the resistor 30 falling within this range. Note that the pressure loss of the resistor 30 can be measured by measuring a difference between the pressure of a fluid at the end surface 30a (inlets of the second flow paths 32) when air in a standard state as the fluid is introduced from the end surface 30a into the resistor 30 at a linear speed of 2.3 mm/sec and the pressure of the fluid at the end surface 30b (outlets of the second flow paths 32).

The ratio of the length of the resistor 30 in the Y1 direction (the length from the end surface 30a to the end surface 30b in the Y1 direction) to the length of the distribution section 20 in the Y1 direction (the length from the introduction port 20a to the lead-out port 20b in the Y1 direction) is preferably equal to or greater than 0.0005%, and is preferably equal to or greater than 0.005%. Also, the ratio of the length of the second reaction section 16B in the Y1 direction to the length of the first reaction section 16A in the Y1 direction is preferably equal to or greater than 50% and equal to or less than 200%, and is more preferably equal to or greater than 100% and equal to or less than 150%. Depolymerization can be appropriately performed, and the decrease in yield of monomer can be appropriately suppressed, by the lengths (heights) of the resistor 30 and the second reaction section 16B in the Y1 direction falling within these ranges.

### (Effects)

The reactor 16 according to the first aspect of the present disclosure includes the reaction section (second reaction section 16B) into which polyester and the reaction solvent M that reacts with the polyester are introduced to depolymerize the polyester and which includes the distribution section 20 and the resistor 30. The distribution section 20 includes the plurality of first flow paths 22 that extend from the introduction port 20a into which the reaction solvent (first solvent M1) from which the first depolymerized polyester P1 as depolymerized polyester has been extracted is introduced to the lead-out port 20b from which the reaction solvent (second solvent M2) from which the second depolymerized polyester P2 as a further depolymerized first depolymerized polyester P1 has been extracted is led out. The resistor 30 is provided at the position at which the resistor 30 blocks the lead-out port 20b of the distribution section 20, and the second flow paths 32 through which the reaction solvent (second solvent M2) flowing through the first flow path 22 is allowed to flow are formed in the resistor 30. The resistor 30 has a higher pressure loss than the distribution section 20.

The reactor 16 of the present disclosure can suppress the decrease in yield of monomer since the first solvent M1 flowing through the second reaction section 16B can be rectified and polyester can be appropriately depolymerized by the second reaction section 16B having the distribution section 20 in which the plurality of first flow paths 22 are formed. Furthermore, the present inventor discovered as a result of intensive studies that the flow of the first solvent M1 is locally accelerated and the retention time of a part of the first solvent M1 is shortened, due to convection of the first solvent M1 occurring in the distribution section 20. If the retention time of the first solvent M1 is shortened, a sufficient time for depolymerization cannot be secured, and the yield of monomer is decreased. On the other hand, the acceleration of the first solvent M1 can be suppressed, and the shortening of the retention time can be suppressed, by providing the resistor 30 at the position at which the lead-out port 20b of the distribution section 20 is blocked in the present disclosure. Therefore, according to the present disclosure, the decrease in yield of monomer can be appropriately suppressed.

In the reactor 16 according to the second aspect of the present disclosure, the resistor 30 preferably blocks the lead-out ports 22b of all the first flow paths 22 in the distribution section 20, in the reactor 16 according to the first aspect. Here, the position where the convection of the first solvent M1 occurs and acceleration occurs in the distribution section 20 may vary depending on an external environment and the like. On the other hand, the acceleration can be appropriately suppressed, and the decrease in yield of monomer can be appropriately suppressed even in the case where the position at which the first solvent M1 is accelerated varies, by providing the resistor 30 at the position at which the lead-out ports 22b of all the first flow paths 22 are blocked.

In the reactor 16 according to the third aspect of the present disclosure, the resistor 30 is preferably provided at a position facing the lead-out port 20b of the distribution section 20, in the reactor 16 according to the first aspect or the second aspect. There becomes no need to insert the resistor 30 into the first flow path 22 by providing the resistor 30 at such a position, and the structure and the assembly operation of the resistor 30 can be simplified. In addition, rectification can be appropriately performed, and the decrease in yield of monomer can be suppressed, by not inserting the resistor 30 into the first flow path 22.

The reactor 16 according to the fourth aspect of the present disclosure preferably further includes a pre-reaction section (first reaction section 16A), in the reactor 16 according to any of the first aspect to the third aspect. The polyester solution P with polyester dissolved therein and the reaction solvent M are introduced into the first reaction section 16A, and the first reaction section 16A brings the polyester solution P into contact with the reaction solvent M, depolymerizes the polyester in the polyester solution P, and extracts the first depolymerized polyester P1 containing the depolymerized polyester into the reaction solvent M. Thus, the polyester can be appropriately depolymerized, and the decrease in yield of monomer can be suppressed.

In the reactor 16 according to the fifth aspect of the present disclosure, the resistor 30 preferably has a higher pressure loss than the first reaction section 16A, in the reactor 16 according to the fourth aspect. Thus, the decrease in yield of monomer can be appropriately suppressed.

In the reactor 16 according to the sixth aspect of the present disclosure, polyester is preferably polyethylene terephthalate, and the reaction solvent M is preferably methanol, in the reactor 16 according to any of the first aspect to the fifth aspect. According to the present disclosure, PET can be appropriately monomerized.

The monomer production system 1 according to the seventh aspect of the present disclosure includes: the reactor 16 according to any of the first aspect to the sixth aspect; and the separation section 18 that is connected to the reactor 16 and separates the reaction solvent (second solvent M2) with the second depolymerized polyester P2 dissolved therein into the reaction solvent M, the monomer D derived from a carboxylic acid contained in the second depolymerized polyester, and the monomer C of an alcohol component contained in the second depolymerized polyester. According to the present disclosure, the decrease in yield of monomer can be appropriately suppressed.

Although the embodiments of the present disclosure have been described above, embodiments are not limited by the content of these embodiments. In addition, the above-described constituent elements include those that can be easily assumed by those skilled in the art, those that are substantially the same, and those in a so-called equivalent range. Furthermore, the above-described constituent elements can be appropriately combined. Furthermore, various omissions, substitutions, or changes of the constituent elements can be made without departing from the gist of the above-mentioned embodiments.

### Reference Signs List

- 10: STORAGE SECTION
- 12: DISSOLUTION SECTION
- 14: SOLVENT STORAGE SECTION
- 16: REACTOR
- 16A: FIRST REACTION SECTION (PRE-REACTION SECTION)
- 16B: SECOND REACTION SECTION (REACTION SECTION)
- 18: SEPARATION SECTION
- D, E: MONOMER
- M: REACTION SOLVENT
- M1: FIRST SOLVENT
- M2: SECOND SOLVENT
- P: POLYESTER SOLUTION
- Pm: POLYESTER RAW MATERIAL
- P1: FIRST DEPOLYMERIZED POLYESTER
- P2: SECOND DEPOLYMERIZED POLYESTER

## Claims

1. A reactor into which polyester and a reaction solvent that reacts with the polyester are introduced to depolymerize the polyester, the reactor comprising a reaction section including:
a distribution section that includes a plurality of first flow paths extending from an introduction port to a lead-out port, the reaction solvent with a first depolymerized polyester as the depolymerized polyester extracted therefrom being introduced into the introduction port, the reaction solvent with a second depolymerized polyester as the further depolymerized first depolymerized polyester extracted therefrom being led out from the lead-out port; and
a resistor that is provided at a position at which the resistor blocks the lead-out port of the distribution section, second flow paths through which the reaction solvent flowing through the first flow paths is allowed to flow being formed in the resistor,
wherein the resistor has a higher pressure loss than the distribution section.

2. The reactor according to claim 1, wherein the resistor blocks the lead-out port at all the first flow paths in the distribution section.

3. The reactor according to claim 1 or 2, wherein the resistor is provided at a position facing the lead-out port of the distribution section.

4. The reactor according to claim 1 or 2, further comprising:
a pre-reaction section into which a polyester solution with the polyester dissolved therein and the reaction solvent are introduced, the pre-reaction section bringing the polyester solution into contact with the reaction solvent to depolymerize the polyester in the polyester solution, the pre-reaction section extracting the first depolymerized polyester containing the depolymerized polyester into the reaction solvent.

5. The reactor according to claim 4, wherein the resistor has a higher pressure loss than the pre-reaction section.

6. The reactor according to claim 1 or 2, wherein the polyester is polyethylene terephthalate, and the reaction solvent is methanol.

7. A monomer production system comprising:
the reactor according to claim 1 or 2; and
a separation section that is connected to the reactor and separates the reaction solvent with the second depolymerized polyester dissolved therein into the reaction solvent, a monomer derived from a carboxylic acid contained in the second depolymerized polyester, and a monomer of an alcohol component contained in the second depolymerized polyester.
